Europäisches Patentamt

(19) European Patent Office

Office européen des brevets

(11) Publication number: **0 195 113**
A2

(12) # EUROPEAN PATENT APPLICATION

(21) Application number: **85109520.8**

(22) Date of filing: **29.07.85**

(51) Int. Cl.⁴: **B 26 F 1/26**
· **B 29 C 51/00, A 41 B 13/02**

(30) Priority: **14.02.85 US 701485**

(43) Date of publication of application:
**24.09.86 Bulletin 86/39**

(84) Designated Contracting States:
**AT BE CH DE FR GB IT LI LU NL SE**

(71) Applicant: **Kargunis, Ltd.**
**13319 Centerville Road**
**Cleveland Wisconsin(US)**

(72) Inventor: **Timmerbeil, Karl E.**
**Severinghauser Strasse 28**
**D-5828 Ennepetal 18(DE)**

(72) Inventor: **Bouda, Francis J.**
**13319 Centerville Road**
**Cleveland Wisconsin 53015(US)**

(74) Representative: **Finck, Dieter et al,**
**Patentanwälte v. Füner, Ebbinghaus, Finck Mariahilfplatz**
**2 & 3**
**D-8000 München 90(DE)**

(54) Textured thermo-plastic film and apparatus for producing the same.

(57) A process and apparatus is described for producing a perforated, textured thermoplastic film (23) having particular application as a cover-stock for disposable, sanitary absorbent products such as baby diapers, sanitary napkins and the like. The apparatus includes a moving metallic belt (32) having perforations therein. The perforations are shaped according to the mathematical formula of an expanded acoustic horn. The film (23) is heated and forced into the perforations so that the inner surface of the projections have the same expanded-horn shape as the perforations. The film (23) may be hydrophyllic with a hydrophobic top surface.

Fig. 3

EP 0 195 113 A2

v. FÜNER    EBBINGHAUS    FINCK

PATENTANWÄLTE    EUROPEAN PATENT ATTORNEYS

MARIAHILFPLATZ 2 & 3, MÜNCHEN 90
POSTADRESSE: POSTFACH 95 01 60, D-8000 MÜNCHEN 95

**0195113**

TITLE MODIFIED
see front page

Kargunis, Ltd.                    EPAC-33054.1
                                  July 29, 1985

## TEXTURED THERMO-PLASTIC FILM

In the field of disposable sanitary absorbent products such as baby diapers, sanitary napkins, adult pads, hospital underpads, protective garments and the like, much effort has been directed to the development of a wrapper which is hydrophobic and which provides a "dry-to-touch" feel.

But it is not enough that the wrapper itself remain dry. It must be constructed so as easily to pass the fluid from the body-contacting side to the inner regions of the absorbent product. It should also function so as to prevent the fluid from moving back to the outer surface. Preferably the inner surface of the wrapper should be spaced from the absorbent core.

The core is usually extremely absorbent (in some constructions a superabsorbent material is used), but if the fluid is not retained within the core and permitted to pass back through the wrapper to the body-contacting outer surface, it ceases to provide the best possible product construction.

Thus, a hydrophobic coverstock material has been sought to replace absorbent cellulose tissue and rayon-fiber materials

which were found acceptable for many years, but which have the undesirable characteristic of keeping the skin of the user "wet" when in use.

During the last decade, coverstock materials have been developed using various polymers, such as polypropylene, polyethylene, polyester, and the like. Extensive work on processes, machinery, and equipment for web forming and bonding synthetic fibers, either with adhesives or by thermo-bonding or entangling have produced spun-bond, thermo bonded, carded, wet laid, dry laid, spun-lace, and melt-blown materials.

The development of perforated film for use as a coverstock material in absorbent disposable sanitary products is not new, and one of the earliest developments is disclosed in German Patent 959814 which issued on March 14, 1957 to Wolfram Weber. This prior art product is illustrated in Figure 10 wherein the water-tight cover of a baby diaper has a plurality of indentations of a generally "tulip" shape with holes at the bottom thereof. The fluid passes from the wearer into the indentations, through the holes, and into the sponge-like absorbing material within the diaper.

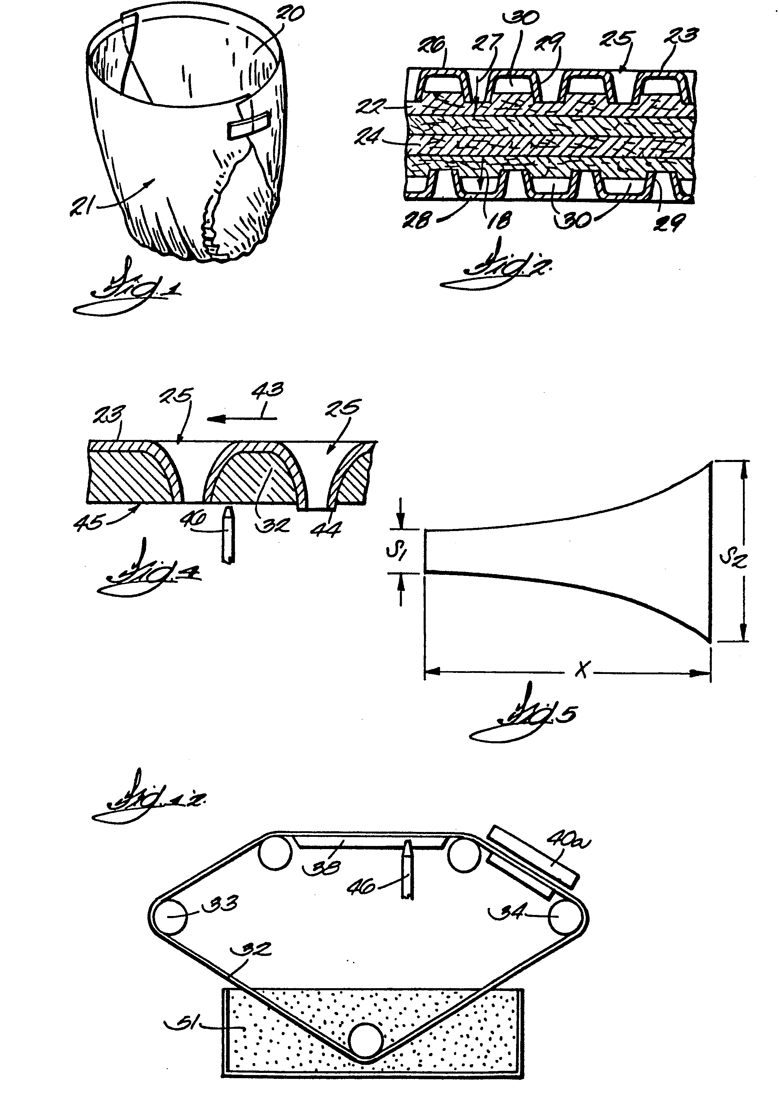

The development of the one-piece baby diaper similar to that shown in Figure 1, was made commercially successful by Procter & Gamble. It is disclosed in the Duncan Patent 3,180,335 (1965) and the subsequent elastic-leg diaper shown in the Buell Patent 3,860,003 (1975).

Between the issuance of Duncan and Buell, Schmedding of Germany secured U.S. Patent 3,628,720 (1971) on a multi-ply

- 3 -

container which utilized an indented portion of one of the plys to hold the plys apart and to provide an air space between. This prior art is illustrated in Figure 7.

In 1974, Kozak of Union Carbide Corporation developed a slit ethylene-ethyl acrylate coverstock material for diapers. It is disclosed in U.S. Patent 3,814,101 (1974) and is illustrated in Figure 8.

A significant breakthrough in the commercialization of perforated film as coverstock material for sanitary disposable absorbent products came with the invention by Thompson of polyethylene film having tapered capillaries or "conulated" elements formed therein. His U.S. Patent 3,929,135 (1975) was commercialized by Procter & Gamble and is illustrated in Figure 6.

Subsequently, Karami of the Colgate-Palmolive Company disclosed in his patent 3,965,909 (1976) how to provide a separate spacer sheet between a perforated coverstock and the absorbent batt of a baby diaper so that the moisture absorbed within the batt would be kept separated from the inner surface of the coverstock sheet material. This prior art is illustrated in Figure 11.

In 1979 a British Patent 2,014,508 issued to Unilever, showing how a coverstock material of a thermoplastic material could be made with right-angle side walls. This is shown in Figure 9.

- 4 -

Furthermore, it is of commercial interest that a perforated plastic film, satisfactory for use as a coverstock material in disposable sanitary absorbent products, can be made and supplied more economically and less expensively than many of the carded webs or spun-bonded or spun-lace material; and, hence, the effort and time expended in developing breathable and porous plastic films are financially rewarding.

A perforated film of the present invention, and the process by which it is made, distinguishes particularly over the prior art in the carefully designed projections and sheet structure which maximizes the flow of liquid therethrough. The design also insures that the the inner surface of the film will be spaced from the absorbent material contained within the wrapper. The projections in the film are trumpet-shaped with precise mathematical and geometrical specification to accomplish this end.

The principle of the preferred and precise shape of the prujections (so as to pass liquid quickly and directly) is the inverse of an exponential acoustic horn.

In an acoustic horn, the fluid (air) is moved efficiently from the throat to the mouth of the horn because of the exponential design.

In the operation of the film of the present invention, the fluid (liquid) deposited at the mouth of the aperture is efficiently moved through the projection to the throat (and hence to the absorbent core) because of the exponential design.

Additionally, the exponential design provides the optimum strength and maximum rigidity of the sides of the projection so as to keep the liquid-bearing core spaced from and coming into contact with the underside of the film.

Furthermore, because the sides of the projection create "pockets" on the inside of the product (as taught by Weber in his German patent 959814) the liquid is trapped within the product and does not move outside to "wet" the wearer's skin.

Therefore, a principal object of the present invention is to provide a process for producing a new and novel perforated film material.

An additional object is to provide a film having apertures with precisely designed trumpet-shaped projections disposed on one side of the film.

With the above and other objects in view, more information and a better understanding of the present invention may be achieved by reference to the following detailed description.

For the purpose of illustrating the invention, there is shown in the accompanying drawings a form thereof which is at present preferred, although it is to be understood that the several instrumentalities of which the invention consists can be variously arranged and organized and that the invention is not limited to the precise arrangements and organizations of the instrumentalities as herein shown and described.

In the drawings, wherein like reference characters indicate like parts:

FIG. 1 is a perspective view of a disposable baby diaper using the perforated film of the present invention.

FIG. 2 is a vertical cross-sectional view of a disposable sanitary pad wrapped in film made according to the present invention.

FIG. 3 is a vertical schematic cross-sectional view of a preferred form of apparatus on which the film of the present invention can be produced on a moving belt.

FIG. 4 is a vertical cross-sectional view, greatly enlarged, of a portion of the belt of Fig. 3, and the film formed thereon.

FIG. 5 is a mathematical representation of the flared trumpet-shaped projection of the perforated film of the present invention.

FIGURES 6-11 are illustrations of prior art devices and films.

FIGURE 12 is a vertical schematic cross-sectional view of a modified form of apparatus of the present invention.

Referring now to FIGURE 1, the top body-contacting sheet 20 of a baby diaper 21 is a particularly effective, end-use for the film of the present invention. The baby diaper 21 and its method of manufacture is well-known in the art and need not be described in detail herein, but is taught in aforesaid U.S. Patents 3,180,335 and 3,860,003 which are made a part hereof and incorporated herein by reference.

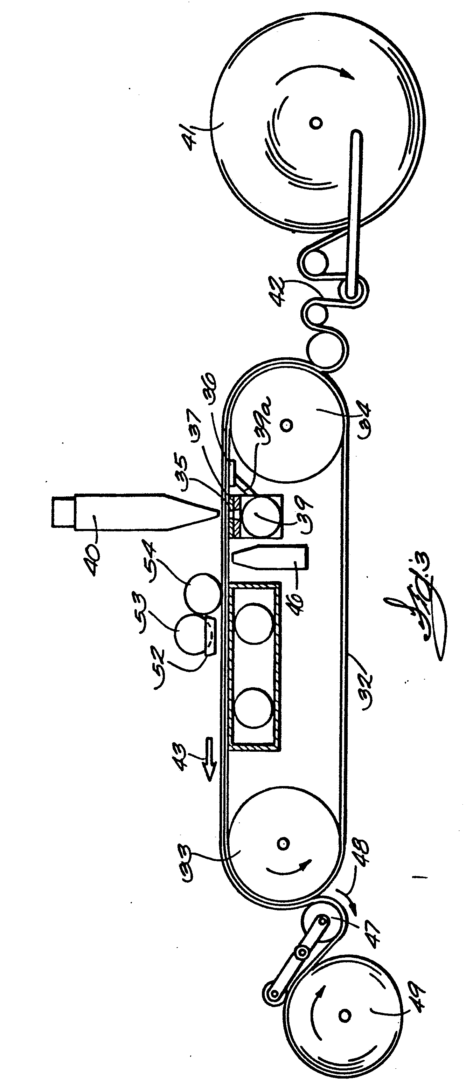

In FIGURE 2, we have shown a cross-section of a sanitary pad 22 having film 23 of the present invention wrapped around a central absorbent core 24. In this form, the apertures 25 on the body-contacting side 26 of pad 22 admit fluid in the direction of the arrow 27 through the projection 29. However, the liquid in the core 24 is prevented from moving out to the back side 28 in the direction of the arrow 29 because it is trapped in the "pockets" 30 formed between the projections 29.

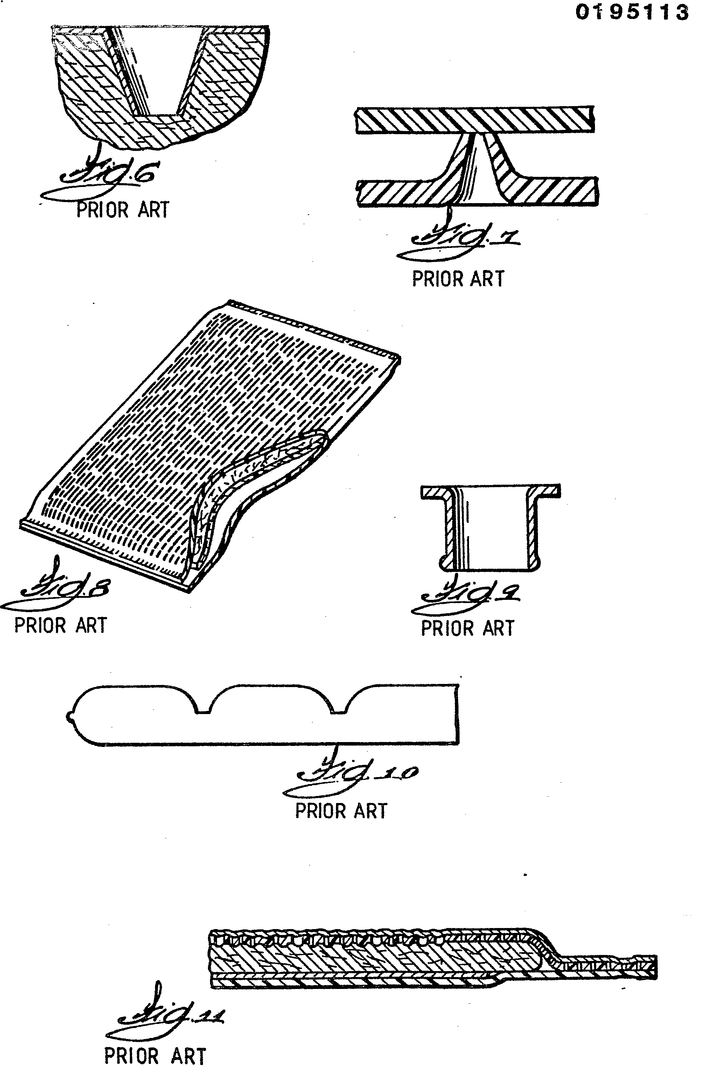

To produce the film 23, we have developed an apparatus 31 shown in FIGURE 3 which includes a belt 32 traveling on appropriate carrier mechanism (not shown in detail), but illustrated between the turning rolls 33 and 34.

The belt 32 has a great many small perforations therein (as many as 1000 per square inch, but preferably between 200 and 500 per square inch), and each perforation has a trumpet-shaped projection.

Appropriate tables 36, 37 and 38 support the belt 32 between the turning rolls 33 and 34 on its upper run.

The principal table 37 has a narrow slot 35 in the upper surface and the slot 35 extends transversely beneath the belt 32 and is in communication with the vacuum chamber 39.

A hot-air jet 40 is positioned above the slot 35 and is directed against the unperforater film 42 as it is carried by the belt 32 from the supply roll 41 across the slot 35. A preferred slot width is 20mm so that the belt 32 does not get drawn into it by the vacuum, but this dimension can vary, depending on the thickness of the belt material.

The table 36 is positioned in advance of the vacuum box 37. The surface of this table 36 may have tiny grooves or be of porous sintered material, connected by the conduit 39-a to the vacuum chamber 39. This design keeps the film 42 in close contact with the belt 32 as the belt approaches the vacuum table 37.

As the unperforated film 42 (which may be a low-density polyethylene web between .5 mils to 2.0 mils, and preferably 1.0 to 1.5 mils thick) is brought to the belt 32 from the roll 41, it is deposited upon the top of the belt 32 in advance of the tables 36 and 37. As the belt moves in the direction of the arrow 43, film 42 is softened by the hot air jet 40, and vacuum in the chamber 39 draws the film into the perforations of the belt (as it passes over the slot 35) in a manner more clearly shown in FIGURE 4.

Any ragged edges 44, which may extend beneath the underside 45 of the belt 32, are undesirable and can be removed as by a flame jet 46.

Thus precisely-formed apertures and projections are formed in the film 23 as the belt 32 is carried toward the turning roll 33. There the film material has cooled sufficiently so that it can be removed from the belt by the take-off roller 47 in the direction of the arrow 48 and wound-up on the roll 49.

Thereafter, the belt continues moving between turning rolls 33 and 34. In the embodiment shown in FIGURE 12, it may pass beneath roller 50 and through a carbon dioxide bath 51 where it is further reduced in temperature.

It is apparent that the belt 32 must be made of materials which withstand the heating and cooling process described, and

to that end we prefer a bolt which is made of etched or electrolytically deposited materials such as nickel or bronze. We prefer nickel because of its good resistance to abrasion. Furthermore, sheets of non-sulfurized nickel can be laser-welded to form a continuous belt. The belt has appropriate holes formed therein to provide a strong, flexible carrier. Because this belt can be made by electrolytic deposition of the metal, the precise configuration of the desired projections, described hereinafter, can be achieved.

In that respect, FIGURE 5 illustrates one form of trumpet-shaped aperture which is provided according to the mathematical formulas for developing an expanded "horn" of the type used in the acoustical field. Such development has not, to the knowledge of the inventors, been heretofore applied in the preparation of a perforated plastic film, or the equipment, or the manufacture thereof, and it is believed that the improvement results in superior liquie handling by the perforated film.

The belt may be made of less flexible materials, such as cast grids, screens, or plates of the types used in the television industry, but because these are relatively stiff, they are made in strip-form and carried between a pair of tracks in a manner similar to the tread on military vehicles. In this form the "belt" can be easily handled and sections of the plates removed and replaced in case of damage or wear, or to provide easily interchangeable indicia or identification or names, etc., which can be formed as a part of the pattern of the holes in such plates. Quite obviously, the cost of replacing individual plates is far less than the cost of replacing a single belt. The plates may be cast 1.7 meters wide, suitable for the width of material used in absorbent pads.

The shape of the horn is determined by a mathematical formula which indicates the ratio of the horn cross-sectional area (at a given distance "x" from the horn throat) to the area of the mouth of the horn. The most widely used horn form is exponential:

$$\frac{S_2}{S_1} = e^{mx}$$

where $S_2$ = mouth area, $S_1$ = throat area, m = flare constant and x = distance from the throat.

In this type of horn, each increment of distance results in a constant ratio of areas: If $S_2 = S_1 (e^{mx})$, then for an increase of x in distance

$$S_2 = S_1 e^{m(x+\_x)} = S_1 (e^{mx} \cdot e^{m\_x})$$

$$= S_1 e^{mx} \cdot e^{m\_x}$$

$$S_2/S_1 = e^{m\_x} \quad \text{is a constant}$$

Other useful horn shapes are Bessel, catenoidal, conical, hyperbolic, and parabolic. These forms are shown in the following illustration:

CYLINDRICAL
1-PARABOLIC
2-CONICAL
3-EXPONENTIAL
4-HYPERBOLIC

The conical shape, the oldest form of artificial horn, is seen in megaphones. The oldest natural form is approximately paraboloidal, similar to the shape form by a pair of cuped hands held before one's mouth. Paraboloidal horns are used, in segments of different flare rates, to approximate horns of other expansion laws, in low-frequency horns. The area of such a section follows a parabolic law of expansion.

The hypobolic and Bessel horns are families of horns, the exact curve depending upon the values of certain parameters. The hyperbolic horn becomes exponential when $T = 1$ and catanoidal when $T = 0$. The Bessel horn becomes conical when $m = 1$.

It is to be understood that the capillary attraction of a drop of liquid in the projection is greater at the throat than at the mouth of the projection and this stronger force draws the droplet through the projection and provides the improved efficiency of our design

In Figure 12 the hot air jet 40 may be replaced by a radiant heater 41-a for films which do not need an air jet to force the film into the apertures in the belt.

We may also provide a finished film 23 which is made of a hydrophyllic base sheet having a hydrophobic coating only on the outer body-contacting surface and not on the inner walls of the projections. To do this we provide above the table 38 a bath 52 of hydrophobic material (such as silicone) which is carried by a transfer roll 53 and printing roll 54 against the outer surface of the film 23 but not into the projections 29. This insures that the body-contacting surface remains "dry-to-touch" and that the liquid is drawn even more efficiently through the trumpet-shaped hydrophyllic projections 29.

In the embodiment shown in Figure 12 the radiant heater 40-a may also be replaced by a corona discharge device to treat the upper surface of the film whereby the surface may be made more acceptable to the silicone coating or to any other process, such as ink printing etc.

The apparatus described above is superior to the drum-type formers disclosed in prior art, such as shown in U.S.Patents 4,214,945 (Lucas,1980), 4,364,723 (Louis,1982),4,441,952 (Mullane,1984) and 4,463,045 (Ahr,1984). The earlier drum-devices all require a squirrel-cage support for the cylindrical plate because it is not possible to make a self-supporting perforated cylinder of such thin material. The smaller the diameter of the holes, the thinner must be the plate material.

Furthermore, the squirrel-cage causes a step-wise vacuum because with every step the gap between the adjacent cage ribs has complete access to the full force of the vacuum. At the initial opening the vacuum is very high, but as soon as the first rows of holes are opened, the vacuum drops down. Conversely, to avoid this loss of suction, a very powerful vacuum pump is required, resulting in a waste of energy. More importantly, the holes are always covered and rendered inoperative where the cage ribs contact the cylindrical plate. The film made on drum machines can usually be identified by the appearance of lines of unopened holes wherever the ribs were present.

The belt of the present invention does not need a squirrel cage support and thus a continuous working perforation system is possible. The belt may be constantly supported by a water-cooled table and only unsupported portion of the belt is where it passes over the vacuum groove, and this is constantly open to the apertures in the belt and thus a constant, non-fluctuating vacuum pulls the film into the apertures in the belt. As mentioned previously, the width of the groove is related to the thickness of the belt material, and can be between 5mm to 20 mm.

It is to be understood that the present invention may be embodied in other specific forms without departing from the spirit or special attributes hereof, and it is therefore desired that the present embodiments be considered in all respects as illustrative, and therefore not restrictive, reference being made to the appended claims rather than to the foregoing description to indicate the scope of the invention.

CLAIMS

Claim 1. In an apparatus for producing perforated thermo-plastic film (23),

- a movable belt (32) having perforations formed therein,
- said perforations each having a mouth, a throat and a distance from throat to mouth and formed according to the formula

$$\frac{S_2}{S_1} = e^{mx}$$

where $S_2$ = mouth area, $S_1$ = throat area, m = flare constant and x = distance from throat toward mouth

- means (41) for laying the film onto the belt,
- means (40) for softening the film while supported on the belt,
- means (39) for forcing the film into the perforations,
- and means (41, 49) for removing the film from the belt.

Claim 2. The apparatus of Claim 1 wherein the means for softening the film (23) is a hot air jet (41).

Claim 3. The apparatus of Claim 1 wherein the means for forcing the film into the perforations is a vacuum box (39) disposed beneath the belt (32).

Claim 4. The apparatus of Claim 3 wherein a narrow slot (35) in the top of the vacuum box (39) is in communication with the perforations in the belt (32).

Claim 5. The apparatus of Claim 1 including means (52, 53, 54) for applying a hydrophobic coating to the top of the film (23) while said film is supported on said belt (32).

Claim 6. Thermo-plastic film having a plurality of apertures (25) therein and projecting portions (29) of said film extending away from one side of said film (23), said projecting portions (29) formed according to the formula:

$$\frac{S_2}{S_1} = e^{mx}$$

Claim 7. The film of Claim 6 wherein the film (23) is formed of a hydrophyllic material and the side of said film opposite said projections is covered with a hydrophobic material.

Claim 8. A sanitary absorbent product having at least a portion thereof covered with a thermo-plastic film made according to Claims 6 or 7.

_Fig.1_

_Fig.2_

_Fig.4_

_Fig.5_

_Fig.3_

_Fig.3_

2/3

0195113

Fig.6
PRIOR ART

Fig.7
PRIOR ART

Fig.8
PRIOR ART

Fig.9
PRIOR ART

Fig.10
PRIOR ART

Fig.11
PRIOR ART